(19) Europäisches Patentamt
European Patent Office
Office européen des brevets

(11) EP 0 944 673 B1

(12) **EUROPÄISCHE PATENTSCHRIFT**

(45) Veröffentlichungstag und Bekanntmachung des Hinweises auf die Patenterteilung:
**13.02.2002 Patentblatt 2002/07**

(21) Anmeldenummer: **97951885.9**

(22) Anmeldetag: **11.11.1997**

(51) Int Cl.$^7$: **C09B 23/10**, B41M 5/38
// G03C7/12, A61K7/13,
G03G9/08

(86) Internationale Anmeldenummer:
**PCT/EP97/06291**

(87) Internationale Veröffentlichungsnummer:
**WO 98/23688 (04.06.1998 Gazette 1998/22)**

(54) **INDOLENINMETHINFARBSTOFFE AUF BASIS VON TRIFLUORMETHYLPYRIDONEN**

TRIFLUORMETHYLPYRIDONE BASED INDOLENINE METHINE DYES

COLORANTS INDOLENINOMETHINIQUES A BASE DE PYRIDONES DE TRIFLUOROMETHYLE

(84) Benannte Vertragsstaaten:
**DE GB IT**

(30) Priorität: **23.11.1996 DE 19648564**
**07.12.1996 DE 19650958**

(43) Veröffentlichungstag der Anmeldung:
**29.09.1999 Patentblatt 1999/39**

(73) Patentinhaber: **DyStar Textilfarben GmbH & Co. Deutschland KG**
**65926 Frankfurt am Main (DE)**

(72) Erfinder:
- **GRUND, Clemens**
**D-68199 Mannheim (DE)**
- **REICHELT, Helmut**
**D-67435 Neustadt (DE)**
- **SCHMIDT, Andreas, Johann**
**D-67251 Freinsheim (DE)**
- **WÜRTHNER, Frank**
**D-89081 Ulm (DE)**
- **SENS, Rüdiger**
**D-68165 Mannheim (DE)**
- **BECKMANN, Stefan**
**D-67098 Bad Dürkheim (DE)**

(56) Entgegenhaltungen:
**DE-A- 4 440 066**
**JP-A- 8 122 972**
**JP-A- 8 171 173**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft neue Pyridonfarbstoffe der Formel I

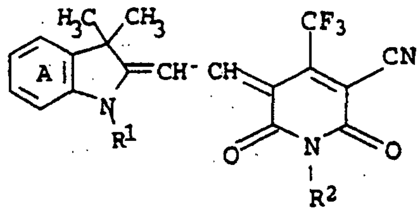

(I),

in der der Ring A substituiert sein kann,

$R^1$ $C_1$-$C_4$-Alkyl, das gegebenenfalls durch Phenyl substituiert ist, und

$R^2$ $C_1$-$C_{13}$-Alkyl, das gegebenenfalls substituiert ist und durch 1 bis 4 Sauerstoffatome in Etherfunktion unterbrochen sein kann, $C_3$-$C_4$-Alkenyl, gegebenenfalls substituiertes Phenyl oder einen Rest der Formel $NE^1E^2$, wobei $E^1$ und $E^2$ gleich oder verschieden sind und unabhängig voneinander jeweils für Wasserstoff, $C_1$-$C_{13}$-Alkyl, das gegebenenfalls substituiert ist und durch 1 bis 3 Sauerstoffatome in Etherfunktion unterbrochen sein kann, $C_5$-$C_7$-Cycloalkyl, gegebenenfalls substituiertes Phenyl, gegebenenfalls substituiertes Pyridyl, gegebenenfalls substituiertes $C_1$-$C_{13}$-Alkanoyl, $C_1$-$C_{13}$-Alkoxycarbonyl, gegebenenfalls substituiertes $C_1$-$C_{13}$-Alkylsulfonyl, $C_5$-$C_7$-Cycloalkylsulfonyl, gegebenenfalls substituiertes Phenylsulfonyl, gegebenenfalls substituiertes Pyridylsulfonyl, gegebenenfalls substituiertes Benzoyl, Pyridylcarbonyl oder Thienylcarbonyl oder $E^1$ und $E^2$ zusammen mit dem sie verbindenden Stickstoffatom gegebenenfalls durch $C_1$-$C_4$-Alkyl substituiertes Succinimido, gegebenenfalls durch $C_1$-$C_4$-Alkyl substituiertes Phthalimido oder einen 5- oder 6-gliedrigen gesättigten heterocyclischen Rest, der gegebenenfalls weitere Heteroatome enthält, stehen, bedeuten, sowie ein Verfahren zu ihrer thermischen Übertragung,

ein Verfahren zum Färben oder Bedrucken von synthetischen Materialien mittels der neuen Farbstoffe sowie ein Verfahren zu ihrem thermischen Transfer.

In der JP-A-339 237/1993 ist die Herstellung von 1-Alkyl-3-cyano-4-trifluormethyl-6-hydroxypyrid-2-onen beschrieben. Weiterhin sind aus der WO-A-96/15 195 Methin- und Azamethinfarbstoffe auf Basis von Trifluormethylpyridonen bekannt.

Aufgabe der vorliegenden Erfindung war es, neue Indoleninmethinfarbstoffe bereitzustellen, die über vorteilhafte anwendungstechnische Eigenschaften bei ihrer textilen Anwendung verfügen.

Demgemäß wurden die eingangs näher bezeichneten Indoleninmethinfarbstoffe der Formel I gefunden.

Alle in den obengenannten Formeln auftretenden Alkyl- oder Alkenylgruppen können sowohl geradkettig als auch verzweigt sein.

Wenn in den obengenannten Formeln substituierte Alkylreste auftreten, so können als Substituenten, sofern nicht anders vermerkt, z.B. Cyclohexyl, gegebenenfalls substituiertes Phenyl, $C_1$-$C_8$-Alkanoyloxy, $C_1$-$C_8$-Alkylaminocarbonyloxy, $C_1$-$C_8$-Alkoxycarbonyl, $C_1$-$C_8$-Alkoxycarbonyloxy, wobei die Alkylkette der drei letztgenannten Reste gegebenenfalls durch 1 bis 3 Sauerstoffatome in Etherfunktion unterbrochen ist und durch Phenyl oder Phenoxy substituiert sein kann, Cyclohexyloxy, Phenoxy, Halogen, Hydroxy, Cyano, Pyrazoyl oder $C_1$-$C_4$-Dialkylamino in Betracht kommen. Die Alkylreste weisen dabei in der Regel 1 oder 2 Substituenten auf.

Wenn in den obengenannten Formeln Alkylreste auftreten, die durch Sauerstoffatome in Etherfunktion unterbrochen sind, so sind, solche Alkylreste bevorzugt, die durch 1 oder 2 Sauerstoffatome in Etherfunktion unterbrochen sind.

Wenn in den obengenannten Formeln substituierte Phenyl- oder Pyridylreste auftreten, so können als Substituenten, ebenso wie im Ring A, z.B. $C_1$-$C_8$-Alkyl, $C_1$-$C_8$-Alkoxy, Halogen, dabei insbesondere Chlor oder Brom, Nitro, Cyano oder $C_1$-$C_4$-Alkoxycarbonyl in Betracht kommen. Die substituierten Reste weisen dabei in der Regel 1 bis 3 Substituenten auf.

Geeignete Reste $R^2$, $E^1$ und $E^2$ sind z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, octyl, 2-Ethylhexyl, Isooctyl, Nonyl, Isononyl, Decyl, Isodecyl, Undecyl, Dodecyl, Tridecyl, Isotridecyl [Die Bezeichnungen Isooctyl, Isononyl, Isodecyl und Isotridecyl sind Trivialbezeichnungen und stammen von den nach der Oxosynthese erhaltenen Alkoholen (vgl. dazu Ullmann's Encyclopedia of Industrial Chemistry, 5th Edition, Vol. A1, Seiten 290 bis 293, sowie Vol. A 10, Seiten 284 und 285).],

2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,9-Trioxadecyl, 3,6,9-Trioxaundecyl, 3,6,9,12-Tetraoxatridecyl, 3,6,9,12-Tetraoxatetradecyl, Phenyl, 2-, 3-oder 4-Methylphehyl. 2-, 3- oder 4-Ethylphenyl, 2-, 3- oder 4-Propylphenyl, 2-, 3- oder 4-Isopropylphenyl, 2-, 3- oder 4-Butylphenyl, 2,4-Dimethylphenyl, 2-, 3- oder 4-Methoxyphenyl, 2-, 3- oder 4-Ethoxyphenyl, 2-, 3- oder 4-Isobutoxyphenyl, 2,4-Dimethoxyphenyl, 2-, 3- oder 4-Chlorphenyl, 2,6-Dichlorphenyl, 2-, 3- oder 4-Nitrophenyl 2-, 3- oder 4-Carboxylphenyl, 2-, 3- oder 4-Cyanophenyl, 2-, 3- oder 4-Methoxy- oder Ethoxycarbonylphenyl, Benzyl, 2-Methylbenzyl, 1- oder 2-Phenylethyl, 2-Hydroxyethyl, 2- oder 3-Hydroxypropyl, 2-Cyanoethyl, 2- oder 3-Cyanopropyl, 2-Acetyloxyethyl, 2- oder 3-Acetyloxypropyl, 2-Isobutyryloxyethyl, 2- oder 3-Isobutyryloxypropyl, 2-Methoxycarbonylethyl, 2- oder 3-Methoxycarbonylpropyl, 2-Ethoxycarbonylethyl, 2- oder 3-Ethoxycarbonylpropyl, 2-Methoxycarbonyloxyethyl, 2- oder 3-Methoxycarbonyloxypropyl, 2-Ethoxycarbonyloxyethyl, 2- oder 3-Ethoxycarbonyloxypropyl, 2-Butoxycarbonyloxyethyl, 2- oder 3-Butoxycarbonyloxypropyl, 2-(2-Phenylethoxycarbonyloxy)ethyl, 2- oder 3-(2-Phenylethoxycarbonyloxy)propyl, 2-(2-Ethoxyethoxycarbonyloxy)ethyl oder 2- oder 3-(2-Ethoxyethoxycarbonyloxy)propyl.

Reste $E^1$ und $E^2$ sind weiterhin z.B. Cyclopentyl, Cyclohexyl oder Cycloheptyl.

Reste $E^1$ und $E^2$ sind weiterhin z.B. Pyridyl, 2-, 3- oder 4-Methylpyridyl, 2-, 3- oder 4-Methoxypyridyl, Formyl, Acetyl, Propionyl, Butyryl, Isobutyryl, Pentanoyl, Hexanoyl, Heptanoyl, Octanoyl, 2-Ethylhexanoyl, Methoxycarbonyl, Ethoxycarbonyl, Propoxycarbonyl, Isopropoxycarbonyl, Butoxycarbonyl, Isobutoxycarbonyl, sec-Butoxycarbonyl, Methylsulfonyl, Ethylsulfonyl, Propylsulfonyl, Isopropylsulfonyl, Butylsulfonyl, Cyclopentylsulfonyl, Cyclohexylsulfonyl, Cycloheptylsulfonyl, Phenylsulfonyl, Tolylsulfonyl, Pyridylsulfonyl, Benzoyl, 2-, 3- oder 4-Methylbenzoyl, 2-, 3- oder 4-Methoxybenzoyl, Thien-2-ylcarbonyl oder Thien-3-yl-carbonyl.

$R^2$ ist weiterhin z.B. Allyl oder Methallyl.

Wenn $E^1$ und $E^2$ zusammen mit dem sie verbindenden Stickstoffatom einen 5- oder 6-gliedrigen gesättigten heterocyclischen Rest, der gegebenenfalls weitere Heteroatome enthältaufweist, bedeuten, so können dafür z.B. Pyrrolidinyl, Piperidinyl, Morpholinyl, Piperazinyl oder N-($C_1$-$C_4$-Alkyl)piperazinyl in Betracht kommen.

Bevorzugt sind Indoleninmethinfarbstoffe der Formel I, in der der Ring A unsubstituiert ist.

Weiterhin bevorzugt sind Indoleninmethinfarbstoffe der Formel I, in der $R^2$ $C_1$-$C_9$-Alkyl, das gegebenenfalls durch 1 bis 3 Sauerstoffatome in Etherfunktion unterbrochen ist und durch Hydroxy oder Phenyl substituiert sein kann, oder Phenyl bedeutet.

Besonders bevorzugt sind Indoleninmethinfarbstoffe der Formel I, in der $R^2$ $C_1$-$C_6$-Alkyl, das gegebenenfalls durch 1 oder 2 Sauerstoffatome in Etherfunktion unterbrochen ist, bedeutet.

Weiterhin bevorzugt sind Indoleninmethinfarbstoffe der Formel I, in der $R^1$ Methyl oder Benzyl bedeutet.

Die erfindungsgemäßen Indoleninmethinfarbstoffe der Formel I können nach an sich bekannten Methoden hergestellt werden.

Beispielsweise können sie durch Kondensation von Aldehyden der Formel II

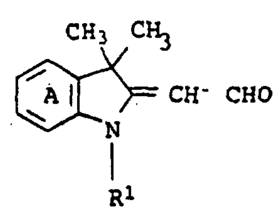

(II),

in der der Ring A und $R^1$ jeweils die obengenannte Bedeutung besitzen, mit Trifluormethylpyridonen der Formel III

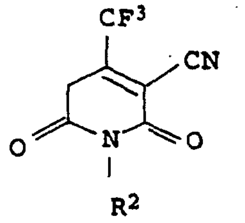

(III)

in der $R^2$ die obengenannte Bedeutung besitzt, erhalten werden.

Bei den Zwischenprodukten für die Herstellung der neuen Indoleninmethinfarbstoffe handelt es sich in der

Regel um an sich bekannte Verbindungen.

Es wurde weiterhin gefunden, daß man synthetische Materialien vorteilhaft färben oder bedrucken kann, wenn man sie mit einem oder mehreren der erfindungsgemäßen Farbstoffe behandelt. Synthetische Materialien sind z.B. Polyester, Polyamide oder Polycarbonate. Insbesondere zu nennen sind Materialien in textiler Form, wie Fasern, Garne, Zwirne, Maschenware, Webware oder Non-wovens aus Polyester, modifiziertem Polyester, z.B. anionisch modifiziertem Polyester, Mischgewebe von Polyester mit Cellulose, Baumwolle, Viskose oder Wolle, oder Polyamid. Die Färbe- und Druckbedingungen sind an sich bekannt und schließen auch das Färben in überkritischem Kohlendioxid mit ein. Man erhält dabei Färbungen oder Drucke mit hoher Lichtechtheit, hoher Brillanz und sehr guten Naßechtheiten, z.B. sehr guter Wasch- oder Schweißechtheit.

Ein weiterer Gegenstand der vorliegenden Erfindung ist ein Verfahren zur Übertragung von Farbstoffen von einem Träger auf ein mit Kunststoff beschichtetes Papier durch Diffusion oder Sublimation mit Hilfe einer Energiequelle, das dadurch gekennzeichnet ist, daß man einen Träger verwendet, auf dem sich ein oder mehrere Indoleninmethinfarbstoffe der Formel I befinden.

Zur Herstellung der für das erfindungsgemäße Verfahren benötigten Farbstoffträger werden die Farbstoffe der Formel I in einem geeigneten organischen Lösungsmittel oder in Mischungen von Lösungsmitteln mit einem oder mehreren Bindemitteln, gegebenenfalls unter Zugabe von Hilfsmitteln zu einer Druckfarbe verarbeitet. Diese enthält die Farbstoffe der Formel I vorzugsweise in molekular-dispers gelöster Form. Die Druckfarbe kann mittels einer Rakel auf den inerten Träger aufgetragen und die Färbung z.B. an der Luft oder mit einem Föhn getrocknet werden. Geeignete organische Lösungsmittel für die Farbstoffe der Formel I sind z.B. solche, in denen die Löslichkeit der Farbstoffe der Formel I bei einer Temperatur von 20°C größer als 1 Gew.-%, vorzugsweise größer als 5 Gew.-% ist.

Beispielhaft seien Ethanol, Propanol, Isobutanol, Tetrahydrofuran, Methylenchlorid, Methylethylketon, Cyclopentanon, Cyclohexanon, Toluol, Chlorbenzol oder deren Mischungen genannt.

Als Bindemittel kommen alle Resins oder Polymermaterialien in Betracht, die in organischen Lösungsmitteln löslich sind und die die Farbstoffe an den inerten Träger abriebfest zu binden vermögen. Dabei werden solche Bindemittel bevorzugt, die die Farbstoffe nach Trocknung der Druckfarbe an der Luft in Form eines klaren, transparenten Films aufnehmen, ohne das dabei eine sichtbare Auskristallisation der Farbstoffe auftritt.

Solche Bindemittel sind beispielsweise in der US-A-5 132 438 oder in den entsprechenden dort zitierten Patentanmeldungen genannt. Darüber hinaus sind gesättigte lineare Polyester zu nennen.

Bevorzugte Bindemittel sind Ethylcellulose, Ethylhydroxyethylcellulose, Polyvinylbutyral, Polyvinylacetat, Cellulosepropionat oder gesättigte lineare Polyester.

Das Gewichtsverhältnis Bindemittel : Farbstoff beträgt im allgemeinen 1 : 1 bis 10 : 1.

Als Hilfsmittel kommen z.B. Trennmittel in Betracht, wie sie in der US-A-5 132 438 oder den entsprechenden dort zitierten Patentanmeldungen genannt sind. Darüber hinaus sind besonders organische Additive zu nennen, welche das Auskristallisieren der Transferfarbstoffe bei Lagerung oder beim Erhitzen des Farbbandes verhindern, z.B. Cholesterin oder Vanillin.

Geeignete inerte Träger sind z.B. in der US-A-5 132 438 oder in den entsprechenden dort zitierten Patentanmeldungen beschrieben. Die Dicke des Farbstoff-Trägers beträgt im allgemeinen 3 bis 30 µm.

Als Farbstoffnehmerschicht kommen prinzipiell alle temperaturstabilen Kunststoffschichten mit Affinität zu den zu transferierenden Farbstoffen in Betracht, z.B. modifizierte Polycarbonate oder Polyester. Weitere Einzelheiten dazu können z.B. aus der US-A-5 132 438 oder den entsprechenden dort zitierten Patentanmeldungen entnommen werden.

Die Übertragung erfolgt mittels einer Energiequelle, z.B. mittels eines Lasers oder eines Thermokopfes, wobei letzterer auf eine Temperatur von $\geq 300°C$ aufheizbar sein muß, damit der Farbstofftransfer im Zeitbereich t: $0 < t < 15$ msec erfolgen kann. Dabei migriert der Farbstoff aus dem Transferblatt und diffundiert in die Oberflächenbeschichtung des Aufnahmemediums.

Die erfindungsgemäßen Farbstoffe der Formel I zeichnen sich beim Farbstofftransfer durch vorteilhafte anwendungstechnische Eigenschaften aus. Sie weisen eine hohe Löslichkeit im Farbband (gute Kompatibilität mit dem Bindemittel), eine hohe Stabilität in der Druckfarbe, eine gute Transferierbarkeit, eine hohe Bildstabilität (d.h. gute Lichtechtheit sowie gute Stabilität gegenüber Umwelteinflüssen, Z.B. Feuchtigkeit, Temperatur oder Chemikalien) auf und erlauben eine flexible coloristische Anpassung an bereits vorgegebene subtraktive Grundfarben im Sinne einer optimalen Trichromie (höchst mögliche Brillanz von Grund- oder Mischfarben und tiefes neutrales Schwarz).

Vorteilhaft eignen sich die neuen Indoleninmethinfarbstoffe auch zum Bedrucken von Materialien mittels des Ink-Jet-Verfahrens. Geeignete Substrate sind dabei neben den obengenannten z.B. Papier, Glas, Keramik, Kunststoffe oder Metalle.

Die erfindungsgemäßen Farbstoffe können auch zum Färben von keratinischen Fasern, z.B. bei der Haarfärbung oder der Färbung von Pelzen, verwendet werden.

Die neuen Farbstoffe der Formel I eignen sich weiterhin vorteilhaft für die Herstellung von Farbfiltern, wie sie z.B. in der EP-A-399 473 beschrieben sind.

Schließlich können sie auch vorteilhaft als Farbmittel für die Herstellung von Tonern für die Elektrophotographie verwendet werden.

Die folgenden Beispiele sollen die Erfindung näher erläutern.

Beispiel 1

a) 100 ml Methanol und 29 g n-Hexylamin (0,287 mol) wurden vorgelegt und bei 30 bis 35 °C innerhalb von 2 h mit 32,8 g (0,287 mol) Cyanessigsäureethylester versetzt. Anschließend rührte man nochmals 2 h bei 30 °C nach. 25 g Piperidin und danach 52,8 g (0,287 mol) 4,4,4-Trifluoracetessigsäureethylester wurden zugegeben. Anschließend erhitzte man die Reaktionsmischung 14 h unter Rückfluß und zog das Lösungsmittel bei einer bis zu 100 °C ansteigenden Temperatur ab. Der noch heiße Rest wurde auf 450 g Eis und 75 ml konz. Salzsäure gegeben. Das Produkt wurde abgesaugt, mit kaltem Wasser neutral gewaschen und bei 50 °C unter vermindertem Druck getrocknet. Man erhielt 79,3 g des Pyridons der Formel

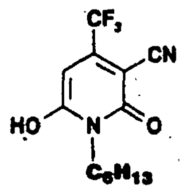

Ausbeute: 95,9 %

b) 4,0 g (0,02 mol) Aldehyd der Formel

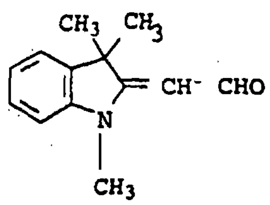

(II),

und 5,8 g (0,02 mol) der in Beispiel 1a) beschriebenen Verbindung wurden in 20 ml Acetanhydrid für 30 min auf 130 °C erhitzt. Nach dem Erkalten fiel ein brillanter roter Farbstoff der Formel

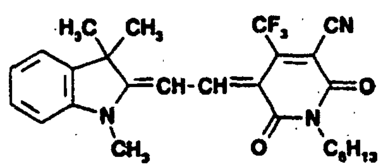

aus. Das Produkt wurde abgesaugt, mit.wenig Methanol gewaschen und bei 50 °C unter vermindertem Druck getrocknet.

Ausbeute: 6,6 g (70 %)
$\lambda_{max}$: 538 nm ($CH_2Cl_2$)

Beispiel 2

a) Zu 210 ml Methanol und 118,3 g 3-(2-Phenoxyethoxy)propylamin (0,6 mol) gab man innerhalb von 2 h bei einer Temperatur von 30 bis 35 °C 67, 9 g (0,6 mol) Cyanessigsäureethylester. Anschließend rührte man nochmals 2 h

bei 30 °C nach. 52,3 g Piperidin und danach 110,4 g (0,6 mol) 4,4,4-Trifluoracetessigsäureethylester wurden zugegeben. Anschließend erhitzte man die Reaktionsmischung 14 h unter Rückfluß und zog das Lösungsmittel bei einer bis zu 100 °C ansteigenden Temperatur ab. Der noch heiße Rest wurde auf 1 l Eis und 157 ml konz. Salzsäure gegeben. Die wäßrige Mischung wurde mit 500 ml Essigester extrahiert, über Natriumsulfat getrocknet und das Lösungsmittel unter vermindertem Druck entfernt. Man erhielt 201 g (87,5 % Ausbeute) des Pyridons der Formel

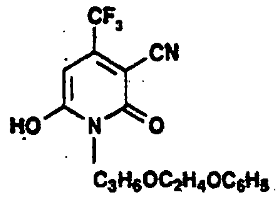

b) 4,0 g (0,02 mol) Aldehyd der Formel

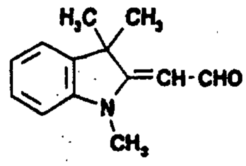

und 7,6 g (0,02 mol) der in Beispiel 2a) beschriebenen Verbindung wurden in 20 ml Acetanhydrid für 30 min auf 130 °C erhitzt. Nach dem Erkalten wurde der ausfallende Farbstoff der Formel

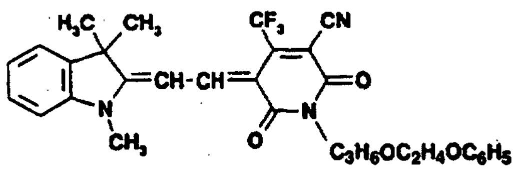

abgesaugt, mit wenig Methanol gewaschen und bei 50 °C unter vermindertem Druck getrocknet.

Ausbeute: 6,25 g (57.8 %)
$\lambda_{max}$: 536 nm ($CH_2Cl_2$)

Beispiel 3

a) Zu 35,4 g (0,6 mol) Propylamin wurden bei 30 bis 35 °C 67,9 g Cyanessigsäureethylester (0,6 mol) zugetropft. Anschließend rührte man 2 h bei 30 °C nach. Nun wurden 52,5 g Piperidin (0,61 mol) und 111,6 g (0,6 mol) 4,4,4-Trifluoracetessigsäureethylester zugetropft. Die Temperatur stieg hierbei auf 53 °C an. Anschließend wurde 10 h unter Rückfluß erhitzt. Das Lösungsmittel wurde am Reaktionsende abdestilliert und der verbleibende Rest heiß in ein Gemisch aus 1 l Eis/Wasser und 160 ml konz. Salzsäure gegossen. Bei 5 °C kristallisierte das Produkt innerhalb 1 h aus, wurde abgesaugt und mit wenig Wasser neutral gewaschen und bei 50 °C unter vermindertem Druck getrocknet. Man erhielt 205 g des Pyridons der Formel

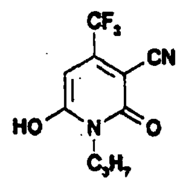

b) 4,0 g Aldehyd (0,02 mol)

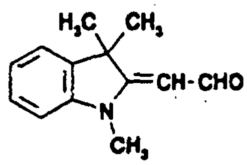

und 4,92 g (0,02 mol) des in Beispiel 3a beschriebenen Pyridons wurden in 20 ml Acetanhydrid für 30 min auf 130 °C erhitzt. Nach dem Erkalten fiel ein roter Farbstoff der Formel

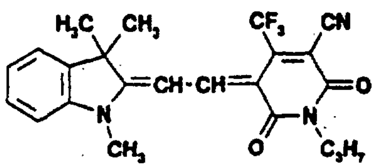

aus, wurde abgesaugt, mit Methanol gewaschen und bei 50 °C unter vermindertem Druck getrocknet.

| | |
|---|---|
| Ausbeute: | 6,5 g (76 %) |
| $\lambda_{max}$: | 536 nm ($CH_2Cl_2$) |

In analoger Weise werden die in der folgenden Tabelle 1 aufgeführten Farbstoffe der Formel

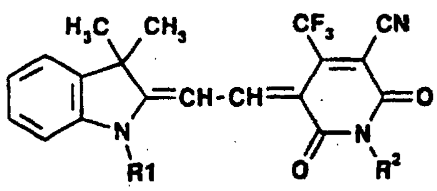

erhalten.

Tabelle 1

| Bsp. Nr. | $R^1$ | $R^2$ | $\lambda_{max}$ [nm] (in $CH_2Cl_2$) |
|---|---|---|---|
| 4 | $CH_3$ | $C_2H_4C_6H_5$ | 536 |
| 5 | $CH_3$ | $CH_2C_6H_5$ | 536 |
| 6 | $CH_3$ | $CH_3$ | 536 |
| 7 | $CH_3$ | $C_4H_9$ | 536 |
| 8 | $CH_3$ | $C_2H_5$ | 536 |
| 9 | $CH_3$ | $(CH_2)_3OCH_3$ | 536 |

| Bsp. Nr. | $R^1$ | $R^2$ | $\lambda_{max}$ [nm] (in $CH_2Cl_2$) |
|---|---|---|---|
| 10 | $CH_3$ | $(CH_2)_2OC_2H_5$ | 536 |
| 11 | $CH_3$ | $(CH_2)_3O(CH_2)_2OCH_3$ | 536 |
| 12 | $CH_3$ | 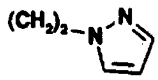 | 536 |
| 13 | $CH_3$ | $(CH_2)_3$-$N(CH_3)_2$ | 536 |
| 14 | $CH_3$ | $C_5H_{11}$ | 537 |
| 15 | $CH_3$ | $CH_2CH(C_2H_5)C_4H_9$ | 536 |
| 16 | $CH_3$ | $C_6H_5$ | 538 |
| 17 | $CH_3$ | $(CH_2)_2OH$ | 536 |
| 18 | $CH_3$ | $(CH_2)_2OCOCH_3$ | 536 |
| 19 | $C_6H_5$ | $C_6H_{13}$ | 537 |
| 20 | $CH_2C_6H_5$ | $C_6H_{13}$ | 536 |
| 21 | $CH_2C_6H_5$ | $C_4H_9$ | 536 |
| 22 | $(CH_2)_2C_6H_5$ | $C_6H_{13}$ | 542 |
| 23 | $(CH_2)_2C_6H_5$ | $C_3H_6OC_2H_4OC_6H_5$ | 542 |
| 24 | $(CH_2)_2C_6H_5$ | $C_3H_7$ | 542 |
| 25 | $(CH_2)_2C_6H_5$ | $C_3H_6OCH_2CH(C_2H_5)C_4H_9$ | 542 |
| 26 | $(CH_2)_2C_6H_5$ | $C_2H_4OC_2H_5$ | 542 |
| 27 | $CH_3$ | $C_3H_6OC_2H_4OC_2H_5$ | 540 |
| 28 | $CH_3$ | $C_3H_6OCH_2CH(C_2H_5)C_4H_9$ | 540 |
| 29 | $C_4H_9$ | $C_4H_9$ | 538 |
| 30 | $C_4H_9$ | $C_3H_6OCH_2CH(C_2H_5)C_4H_9$ | 540 |
| 31 | $CH_3$ | $C_3H_6OCH_2C_6H_5$ | 538 |
| 32 | $CH_3$ | $C_2H_4OC_6H_4$-p-$OCH_3$ | 538 |
| 33 | $CH_3$ | $CH(CH_3)C_3H_6CH(CH_3)CH_3$ | 538 |
| 34 | $CH_3$ | $C_3H_6OC_3H_7$ | 538 |

Anwendung beim Färben

10 g Polyestergewebe werden bei einer Temperatur von 50°C in 200 ml einer Färbeflotte gegeben, die X Gew.-%, bezogen auf das Polyestergewebe, Farbstoff enthält und deren PH-Wert mittels Essigsäure auf 4,5 eingestellt ist. Man behandelt 5 min bei 50°C, steigert dann die Temperatur der Flotte innerhalb von 30 min auf 130°C, hält 60 min bei dieser Temperatur und läßt dann innerhalb von 20 min auf 60°C abkühlen.

Danach wird das ausgefärbte Polyestergewebe reduktiv gereinigt, indem man es 15 min in 200 ml einer Flotte,

die 5 ml/l 32 gew.-%ige Natronlauge, 3 g/l Natriumdithionit und 1 g/l eines Anlagerungsproduktes von 48 mol Ethylenoxid an 1 mol Ricinusöl enthält, bei 65°C behandelt. Schließlich wird das Gewebe gespült, mit verdünnter Essigsäure neutralisiert, nochmals gespült und getrocknet.

Die Farbstoffe Nr. 1 bis 34 wurden jeweils in einer Menge (X) von 0,3 Gew.-% angewandt. Man erhielt hochbrillante rote Färbungen mit hervorragender Lichtechtheit.

Anwendung in der thermischen Übertragung:

a) 10 g Farbstoff werden, gegebenenfalls unter kurzzeitigem Erwärmen auf 80 bis 90°C, in 100 g einer 10 gew.-%igen Lösung eines Bindemittels auf Basis von Polyvinylbutyral in einem Methylethylketon/Toluol/Cyclohexanon-Gemisch (4,5:2:2 v/v/v) eingerührt.

Das Gemisch wird mit einer 10 µm Rakel auf eine Polyesterfolie von 6 µm Dicke, auf deren Rückseite eine geeignete Gleitschicht aufgebracht ist, aufgerakelt und mit einem Föhn 1 Minute trockengeblasen. Bevor das Farbband verdruckt werden kann, muß es mindestens 24 Stunden an der Luft nachtrocknen, da Restlösungsmittel den Druckvorgang beeinträchtigen können.

b) Die Farbbänder werden auf einer rechnergesteuerten Versuchsanordnung, die mit einem handelsüblichen Thermokopf ausgestattet ist, auf handelsübliches Videoprintpapier (Color Videoprint Paper der Firma Hitachi) verdruckt.

Durch Veränderung der Spannung wird die Energieabgabe des Thermokopfs gesteuert, wobei die eingestellte Impulsdauer 7 ms beträgt und immer nur ein Impuls abgegeben wird. Die abgegebene Energie liegt zwischen 0,5 und 2,0 mJ/Dot.

Da die Höhe der Anfärbung direkt proportional der zugeführten Energie ist, kann ein Farbkeil erzeugt und spektroskopisch ausgewertet werden.

Aus der graphischen Auftragung der Farbtiefe gegen die zugeführte Energie je Heizelement wird der Q*-Wert (= Energie in mJ für den Extinktionswert 1) und die Steigung m in 1/mJ ermittelt.

Die erhaltenen Ergebnisse sind in der folgenden Tabelle 2 aufgeführt.

Tabelle 2

| Farbstoff Nr. | Q* [$\frac{mJ}{Dot}$] | m [$\frac{1}{mJ}$] |
|---|---|---|
| 1 | 0,93 | 2,89 |
| 2 | 0,94 | 2,54 |
| 4 | 0,98 | 2,65 |
| 5 | 1,00 | 2,25 |
| 7 | 0,93 | 2,85 |
| 11 | 0,91 | 2,84 |
| 15 | 0,98 | 2,67 |

## Patentansprüche

1. Indoleninmethinfarbstoffe der Formel I

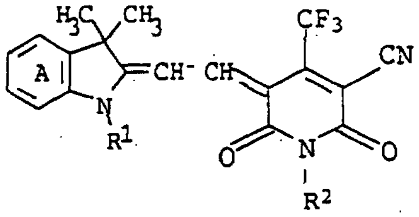

(I),

in der
der Ring A substituiert sein kann,

$R^1$ $C_1$-$C_4$-Alkyl, das gegebenenfalls durch Phenyl substituiert ist, und

$R^2$ $C_1$-$C_{13}$-Alkyl, das gegebenenfalls substituiert ist und durch 1 bis 4 Sauerstoffatome in Etherfunktion unterbrochen sein kann, $C_3$-$C_4$-Alkenyl, gegebenenfalls substituiertes Phenyl oder einen Rest der Formel $NE^1E^2$, wobei $E^1$ und $E^2$ gleich oder verschieden sind und unabhängig voneinander jeweils für Wasserstoff, $C_1$-$C_{13}$-Alkyl, das gegebenenfalls substituiert ist und durch 1 bis 3 Sauerstoffatome in Etherfunktion unterbrochen sein kann, $C_5$-$C_7$-Cycloalkyl, gegebenenfalls substituiertes Phenyl, gegebenenfalls substituiertes Pyridyl, gegebenenfalls substituiertes $C_1$-$C_{13}$-Alkanoyl, $C_1$-$C_{13}$-Alkoxycarbonyl, gegebenenfalls substituiertes $C_1$-$C_{13}$-Alkylsulfonyl, $C_5$-$C_7$-Cycloalkylsulfonyl, gegebenenfalls substituiertes Phenylsulfonyl, gegebenenfalls substituiertes Pyridylsulfonyl, gegebenenfalls substituiertes Benzoyl, Pyridylcarbonyl oder Thienylcarbonyl oder $E^1$ und $E^2$ zusammen mit dem sie verbindenden Stickstoffatom gegebenenfalls durch $C_1$-$C_4$-Alkyl substituiertes Succinimido, gegebenenfalls durch $C_1$-$C_4$-Alkyl substituiertes Phthalimido oder einen 5- oder 6-gliedrigen gesättigten heterocyclischen Rest, der gegebenenfalls weitere Heteroatome enthält, stehen, bedeuten.

2. Indoleninmethinfarbstoffe nach Anspruch 1, **dadurch gekennzeichnet, daß** der Ring A unsubstituiert ist.

3. Indoleninmethinfarbstoffe nach Anspruch 1, **dadurch gekennzeichnet, daß** $R^2$ $C_1$-$C_9$-Alkyl, das gegebenenfalls durch 1 bis 3 Sauerstoffatome in Etherfunktion unterbrochen ist und durch Hydroxy oder Phenyl substituiert sein kann, oder Phenyl bedeutet.

4. Verfahren zum Färben oder Bedrucken von synthetischen Materialien, **dadurch gekennzeichnet, daß** man die synthetischen Materialien mit einem oder mehreren Indoleninmethinfarbstoffen gemäß Anspruch 1 behandelt.

5. Verfahren zur Übertragung von Farbstoffen von einem Träger auf ein mit Kunststoff beschichtetes Papier durch Diffusion oder Sublimation mit Hilfe einer Energiequelle, **dadurch gekennzeichnet, daß** man einen Träger verwendet, auf dem sich ein oder mehrere Indoleninmethinfarbstoffe der Formel I gemäß Anspruch 1 befinden.

## Claims

1. Indoleninemethine dyes of the formula I

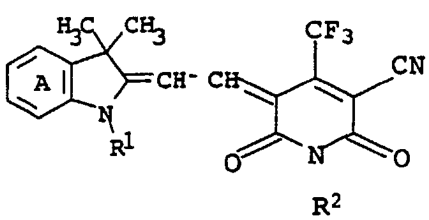

(I),

where
the ring A is substituted or unsubstituted,

$R^1$ is $C_1$-$C_4$-alkyl with or without substitution by phenyl, and

$R^2$ is $C_1$-$C_{13}$-alkyl with or without substituion and with or without interruption by from 1 to 4 oxygen atoms in ether function, $C_3$-$C_4$-alkenyl, substituted or unsubstituted phenyl or a radical of the formula $NE^1E^2$, where $E^1$ and $E^2$ are identical or different and each is independently of the other hydrogen, $C_1$-$C_{13}$-alkyl with or without substitution and with or without interruption by from 1 to 3 oxygen atoms in ether function, $C_5$-$C_7$-cycloalkyl, substituted or unsubstituted phenyl, substituted or unsubstituted pyridyl, substituted or unsubstituted $C_1$-$C_{13}$-alkanoyl, $C_1$-$C_{13}$-alkoxycarbonyl, substituted or unsubstituted $C_1$-$C_{13}$-alkylsulfonyl, $C_5$-$C_7$-cycloalkylsulfonyl, substituted or unsubstituted phenylsulfonyl, substituted or unsubstituted pyridylsulfonyl, substituted or unsubstituted benzoyl, pyridylcarbonyl or thienylcarbonyl, or $E^1$ and $E^2$ join with the linking nitrogen atom to form unsubstituted or $C_1$-$C_4$-alkyl-substituted succinimido, unsubstituted or $C_1$-$C_4$-alkyl-substituted phthalimido or a 5- or 6-membered saturated heterocyclic radical with or without further hetero atoms.

2. Indoleninemethine dyes as claimed in claim 1, wherein the ring A is unsubstituted.

3. Indoleninemethine dyes as claimed in claim 1, wherein $R^2$ is $C_1$-$C_9$-alkyl with or without interruption by from 1 to 3 oxygen atoms in ether function and with or without hydroxyl or phenyl substitution or phenyl.

4. A process for dyeing or printing synthetic materials, which comprises treating the synthetic materials with one or more indoleninemethine dyes as claimed in claim 1.

5. A process for transferring dyes from a transfer to polymer-coated paper by diffusion or sublimation with the aid of an energy source, which comprises using a transfer comprising one or more indoleninemethine dyes of the formula I as set forth in claim 1.

**Revendications**

1. Colorants indoléninométhiniques de formule I

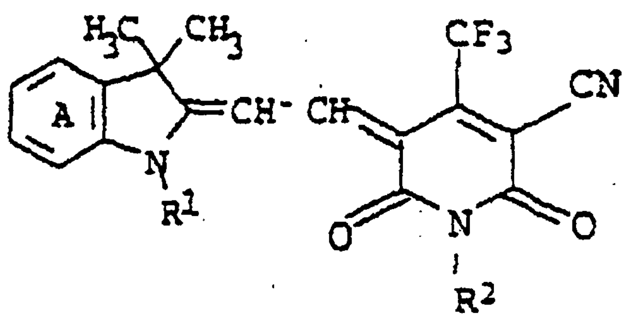

(I),

dans laquelle
le cycle A peut être substitué,

$R^1$ représente un groupe alkyle en $C_1$-$C_4$ qui est éventuellement substitué par le groupe phényle, et
$R^2$ représente un groupe alkyle en $C_1$-$C_{13}$ qui est éventuellement substitué et peut être interrompu par 1 à 4 atomes d'oxygène en fonction éther, un groupe alcényle en $C_3$-$C_4$, un groupe phényle éventuellement substitué ou un radical de formule $NE^1E^2$, $E^1$ et $E^2$ étant identiques ou différents et représentant chacun, indépendamment l'un de l'autre, un atome d'hydrogène, un groupe alkyle en $C_1$-$C_{13}$ qui est éventuellement substitué et peut être interrompu par 1 à 3 atomes d'oxygène en fonction éther, un groupe cycloalkyle en $C_5$-$C_7$, un groupe phényle éventuellement substitué, un groupe pyridyle éventuellement substitué, un groupe alcanoyle en $C_1$-$C_{13}$ éventuellement substitué, un groupe alcoxy($C_1$-$C_{13}$)carbonyle, un groupe alkyl($C_1$-$C_{13}$) sulfonyle éventuellement substitué, un groupe cycloalkyl($C_5$-$C_7$)-sulfonyle, un groupe pyridylsulfonyle éventuellement substitué, un groupe benzoyle éventuellement substitué, pyridylcarbonyle ou thiénylcarbonyle, ou $E^1$ et $E^2$ représentent ensemble, avec l'atome d'azote qui les relie, un groupe succinimido éventuellement substitué par un groupe alkyle en $C_1$-$C_4$, un groupe phtalimido éventuellement substitué par un groupe alkyle en $C_1$-$C_4$, ou un radical hétérocyclique saturé à 5 ou 6 chaînons, qui comporte éventuellement d'autres hétéroatomes.

2. Colorants indoléninométhiniques selon la revendication 1, **caractérisés en ce que** le cycle A est non substitué.

3. Colorants indoléninométhiniques selon la revendication 1, **caractérisés en ce que** $R^2$ représente un groupe alkyle en $C_1$-$C_9$ qui est éventuellement interrompu par 1 à 3 atomes d'oxygène en fonction éther et peut être substitué par le groupe hydroxy ou phényle, ou représente le groupe phényle.

4. Procédé pour la teinture ou l'impression de matériaux synthétiques, **caractérisé en ce qu'**on traite les matériaux synthétiques par un ou plusieurs colorants indoléninométhiniques selon la revendication 1.

5. Procédé pour le transfert de colorants d'un support sur un papier revêtu de matière synthétique, par diffusion ou

sublimation à l'aide d'une source d'énergie, **caractérisé en ce qu'**on utilise un support sur lequel se trouve(nt) un ou plusieurs colorants indoléninométhiniques de formule I selon la revendication 1.